# EUROPÄISCHE PATENTSCHRIFT

(11) **EP 0 637 432 B1**
(45) Veröffentlichungstag und Bekanntmachung des Hinweises auf die Patenterteilung: **01.10.1997**
(21) Anmeldenummer: 94111139.5
(22) Anmeldetag: 18.07.1994
(51) Int. Cl.: A61B 17/12

(54) **Umfahrungsinstrument**
Looping instrument
Instrument pour contourner

(30) Priorität: 03.08.1993 DE 4325969
(43) Veröffentlichungstag der Anmeldung: 08.02.1995
(73) Patentinhaber: AESCULAP AG, 78532 Tuttlingen (DE)
(72) Erfinder: Giordano, Nicola, D-78056 Villingen-Schwenningen (DE); Weisshaupt, Dieter, D-78194 Immendingen (DE); Wieneke, Paul, D-78604 Rietheim-Weilheim (DE)
(74) Vertreter: Hoeger, Stellrecht & Partner

(56) Entgegenhaltungen:
- WO-A-90/06725
- DE-C- 4 133 800
- US-A- 4 403 985

## Beschreibung

Die Erfindung betrifft ein Umfahrungsinstrument mit einem um einen Gegenstand herumlegbaren, länglichen, streckbaren und abbiegbaren Arm, an dessen freiem Ende ein Haltewerkzeug angeordnet ist.

An schwer zugänglichen Stellen ist es schwierig, einen Gegenstand zu umfahren, beispielsweise um einen Faden um den Gegenstand zu legen oder um den Gegenstand mit dem Umfahrungsinstrument zu verschieben. Dieses Problem stellt sich beispielsweise bei der Chirurgie, wenn schwer zugängliche Körperteile abgebunden, gehalten oder verlagert werden sollen. Bei der laparoskopischen Operationsmethode wird durch einen kleinen Einschnitt in der Bauchdecke des Patienten im Körperinneren operiert, und dabei müssen beispielsweise Blutgefäße abgebunden oder Darmschlingen umfahren werden. Die dazu erforderlichen Instrumente müssen schlank und länglich sein, da sie durch die kleine Körperöffnung in den Körper eingeführt werden müssen, trotzdem müssen sie an jeder Stelle im Innenraum des Körpers handbabbar sein und die zum Teil sehr kleinen Körperteile umschlingen können.

Es ist dazu aus DE-C-4133800 ein Instrument bekannt, bei dem eine abgebogene Feder an ihrem freien Ende eine Fadenöse trägt. Diese Feder kann mehr oder weniger tief in ein geradliniges Rohr eingezogen werden, so daß die Feder dabei gestreckt wird. Wird sie aus dem Rohr ausgefahren, kann sie ihre natürliche Krümmung wieder einnehmen. Bei geschickter Handhabung ist es möglich, eine solche Feder um ein Körperteil herumzulegen, jedoch ist man einerseits an die natürliche Krümmung dieser Feder gebunden, da die Krümmung nicht verändert werden kann, andererseits ist die Handhabbarkeit schwierig, da zum Abbiegen der Feder die Hülse in axialer Richtung verschoben werden muß.

Es ist Aufgabe der Erfindung, ein Umfahrungsinstrument der gattungsgemäßen Art so auszubilden, daß die Handhabbarkeit verbessert wird, insbesondere durch die willkürliche Veränderung des Biegeradius und des Umfahrungwinkels.

Diese Aufgabe wird bei einem Umfahrungsinstrument der eingangs beschriebenen Art erfindungsgemäß dadurch gelöst, daß der Arm mindestens über einen Teil seiner Länge als Schlauch aus einem dehnbaren Material ausgebildet ist, dessen Längsdehnbarkeit auf zwei einander gegenüberliegenden Seiten verschieden ist, daß das Innere des Schlauches mit einer ein fließfähiges Medium unter Druck in den Schlauch einfüllenden Druckquelle verbunden ist und daß der Arm im schlauchförmigen Bereich mindestens um 180° abbiegbar ist.

Bei dem neuen Instrument wird als biegbarer Arm also ein schlauch- oder katheterförmiges Element verwendet, das durch Befüllung mit einer fließfähigen Flüssigkeit, beispielsweise einer Ringerlösung, in dem dehnbaren Bereich steuerbar abgebogen werden kann. Der Abbiegungswinkel hängt dabei vom Füllungsgrade einerseits und von der Länge des dehnbaren Schlauchbereiches andererseits ab, so daß bei der Handhabung eines bestimmten Instrumentes durch unterschiedliche Befüllung feinfühlig Umschlingungswinkel und Biegeradius variiert werden können, ohne daß dazu eine mechanische Verschiebung an dem Arm notwendig wäre, wie bei dem vorbekannten Federinstrument.

Es ist daher möglich, den Arm um ein beliebiges Teil oder einen beliebigen Gegenstand herumzulegen, beispielsweise um ein Blutgefäß.

Bei einer ersten bevorzugten Ausführungsform kann vorgesehen sein, daß das Haltewerkzeug ein Fadenhalter ist, insbesondere in Form eines Hakens. Es ist damit möglich, einen Faden um einen Gegenstand herumzulegen, beispielsweise einen Ligaturfaden um ein Blutgefäß. Bei einer bevorzugten Ausführungsform ist vorgesehen, daß längs des Armes ein Fadenkanal angeordnet ist, in dem also der Faden geführt ist. Dabei kann der Fadenkanal im Innern des Armes angeordnet sein, bei einer anderen Ausführungsform wird der Fadenkanal durch eine Rinne oder Einstülpung in der Wand des Armes gebildet.

Während es bei einem Umfahrungsinstrument, mit dem ein Faden um einen Gegenstand herumgelegt werden soll, genügen kann, wenn der Arm des Instrumentes um 180° abbiegbar ist, so daß man den Faden dann mit einem anderen Instrument auf beiden Seiten des Gegenstandes fassen kann, ist es bei einer bevorzugten Ausführungsform des Umfahrungsinstrumentes günstig, wenn der Arm so weit abbiegbar ist, daß sein freies Ende den rückwärtigen Teil des Armes erreicht, wenn also eine vollständige Schlinge gebildet wird.

Dabei kann beispielsweise vorgesehen sein, daß das Haltewerkzeug ein Haken ist, der so dimensioniert ist, daß er um einen rückwärtigen Bereich des Armes legbar ist. Es ist dadurch möglich, mit dem Arm eine den zu umfahrenden Gegenstand umgebende Schlinge zu bilden, die durch Zug an dem Arm auch fest geschlossen werden kann. Man kann auf diese Weise ohne Verwendung eines Ligaturfadens den Umfahrungsarm selbst als Ligaturelement verwenden, das unter Umständen nach dem Anlegen auch von der Druckquelle abgetrennt werden kann.

Bei einem solchen, eine vollständige Schlinge ausbildenden Umfahrungsinstrument ist es notwendig, zum Eingreifen des Hakens im rückwärtigen Bereich des Armes den Haken oder den rückwärtigen Bereich des Armes senkrecht zu der von der Schlinge ausgebildeten Ebene zu bewegen, so daß der Haken seitlich um den rückwärtigen Teil des Armes greifen kann. An schwer zugänglichen Stellen ist dies unter Umständen schwer durchführbar, so daß es günstig ist, wenn bei einer weiteren Ausgestaltung eines solchen Umfahrungswerkzeuges im Innern des schlauchförmigen Teiles des Armes mehrere getrennt befüllbare Kammern angeordnet sind, so daß durch deren gezielte Befüllung ein Abbiegen des Schlauches nach verschiedenen Richtungen möglich ist. Beispielsweise können über den Umfang drei solche Kammern verteilt sein, die bei entsprechend gezielter Befüllung ein Abbiegen des schlauchförmigen Teiles in eine beliebige Richtung ermöglichen. Dadurch kann das freie Ende des Umfahrungswerkzeuges auch senkrecht zur Ebene der ausgebildeten Schlinge seitlich an den rückwärtigen Teil des Armes herangeführt werden, so daß der Haken am freien Ende den rückwärtigen Teil umgreift. In ähnlicher Weise kann auch eine Lösung der angelegten Schlinge erfolgen.

Die nachfolgende Beschreibung bevorzugter Ausführungsformen der Erfindung dient im Zusammenhang mit der Zeichnung der näheren Erläuterung. Es zeigen:
- Figur 1:: eine einfache Ausführungsform eines als Fadenanlegers verwendeten Umfahrungsinstrumentes;
- Figur 2:: den vorderen Armteil bei einem anderen Ausführungsbeispiel eines als Fadenanleger verwendeten Umfahrungsinstrumentes;
- Figur 3:: eine Schnittansicht längs Linie 3-3 in Figur 2;
- Figur 4:: eine Ansicht ähnlich Figur 2 bei einem als Umschlingungsinstrument verwendeten Umfahrungsinstrument und
- Figur 5:: eine Schnittansicht längs Linie 5-5 in Figur 4.

Das in der Zeichnung dargestellte Umfahrungsinstrument wird am Beispiel eines Umfahrungsinstrumentes erläutert, das zu chirurgischen Zwecken eingesetzt wird, beispielsweise in der Laparoskopie. Es handelt sich dabei um eine bevorzugte Verwendung eines solchen Umfahrungsinstrumentes, jedoch versteht es sich, daß ein solches Umfahrungsinstrument auch für andere Zwecke verwendet werden kann, beispielsweise zur Handhabbarkeit von Teilen einer elektrischen Schaltung etc.

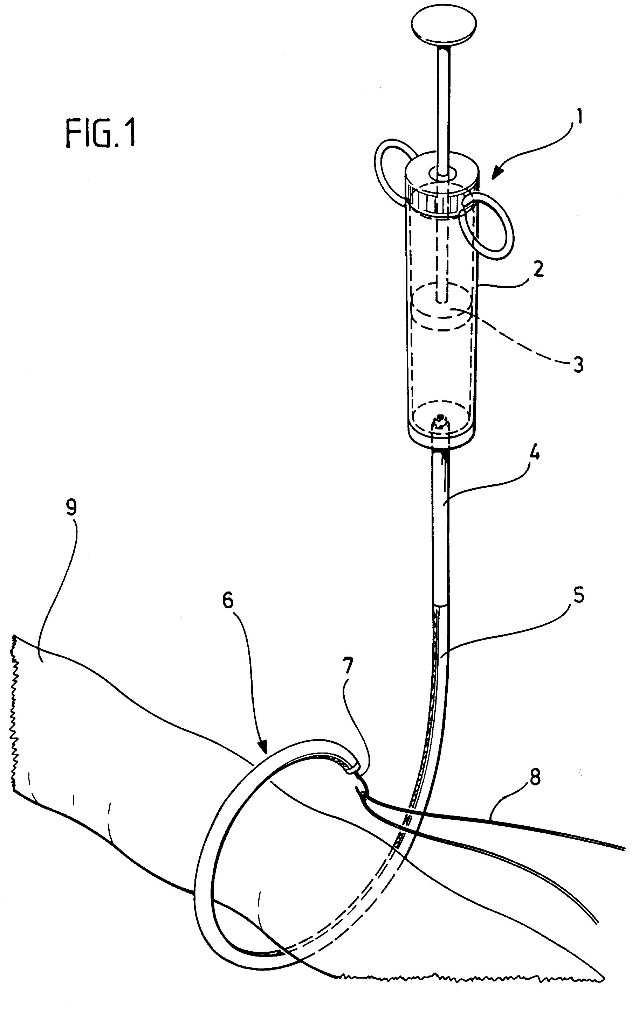

Das in Figur 1 dargestellte Umfahrungsinstrument umfaßt einen Spritzenkörper 1 mit einem Aufnahmebehälter 2 für eine Flüssigkeit, beispielsweise eine Ringerlösung, und einen in diesem Aufnahmebehälter 2 abgedichtet verschiebbaren Kolben 3, durch den Flüssigkeit aus dem Aufnahmebehälter 2 in ein aus dem Aufnahmebehälter 2 austretendes Rohr 4 gedrückt werden kann. An dieses Rohr 4, das starr ausgebildet sein kann, schließt sich ein flexibler Schlauch 5 an, der an das starre Rohr 4 angeformt oder auf dieses aufgesteckt sein kann, dieser Schlauch 5 bildet einen abbiegbaren Arm 6 des Umfahrungsinstrumentes. Der Schlauch 5 ist an seinem freien Ende verschlossen, sein Innenraum steht mit dem Aufnahmebehälter 2 in Verbindung, so daß der Schlauch gefüllt wird, wenn mittels des Kolbens 3 Flüssigkeit aus dem Aufnahmebehälter 2 verdrängt wird.

Der Schlauch 5 besteht aus einem in Längsrichtung dehnbaren Material, beispielsweise aus Silikon, seine Längsdehnbarkeit ist längs des Umfanges verschieden. Dies kann beispielsweise dadurch erreicht werden, daß der Innenraum des Schlauches exzentrisch angeordnet ist oder daß an einer Seite des Schlauches 5 eine die Längenausdehnung verhindernde Wandverstärkung angeordnet ist, zum Beispiel in Form eines in die Schlauchwand eingebetteten, in Längsrichtung nicht dehnbaren Fadens. Diese in Umfangsrichtung unterschiedliche Dehnbarkeit führt dazu, daß bei einer Füllung des Schlauches 5 mit der Lösung die Schlauchwand an einer Seite gedehnt und an der gegenüberliegenden Seite weniger oder gar nicht gedehnt wird. Insgesamt erhält man dadurch eine Abbiegung des Schlauches nach der weniger gedehnten Seite. Diese Abbiegung hält an, solange der Druck im Innern des Schlauches erhöht ist, wenn der Druck wieder erniedrigt wird, kehrt der Schlauch aufgrund der eigenen Elastizität des Materials wieder in seine gestreckte Ausgangslage zurück.

Der Schlauch muß nicht über seine gesamte Länge in dieser Weise dehnbar ausgebildet sein, es kann vorgesehen sein, daß nur ein bestimmter Bereich des Schlauches in dieser Weise längsdehnbar und damit abbiegbar ist, während andere Schlauchbereiche in Längsrichtung nicht dehnbar ausgebildet sind, beispielsweise dadurch, daß um den Schlauch in dem fraglichen Bereich eine Manschette gelegt wird, etwa ein Gewebemantel. Es ist auch möglich, über die Länge des Schlauches unterschiedliche Krümmungen dadurch zu erzeugen, daß die Unterschiede der Längsdehnbarkeit auf gegenüberliegenden Seiten des Schlauches verschieden ausgeführt werden. Man kann dann beispielsweise beim Befüllen des Schlauches zum freien Ende hin eine immer enger werdende Krümmung des Schlauches erreichen.

Am freien Ende trägt der Arm im Ausführungsbeispiel der Figur 1 einen einseitig offenen Haken 7, in den ein Faden 8 eingelegt ist. Das beschriebene Umfahrungsinstrument, das beispielsweise durch einen kleinen Einschnitt in der Bauchdecke in den Körperinnenraum eingeführt werden kann, wird zusammen mit dem im Haken 7 eingelegten Faden 8 zunächst einseitig an einem zu umfahrenden Körperteil vorbeigeschoben, beispielsweise an einem Blutgefäß 9, anschließend wird der Schlauch 5 durch Befüllung des Schlauches mit der Lösung so weit abgebogen, daß der Arm den zu umfahrenden Körperteil umschlingt und dabei den Faden 8 gleichfalls um diesen Körperteil herumlegt.

Es kann auch in der Weise vorgegangen werden, daß zunächst der Arm 6 ohne eingelegten Faden 8 um das Körperteil herumgelegt wird, anschließend wird der Faden 8 in den Haken 7 eingelegt, und anschließend wird der Schlauch 5 wieder entleert und von dem umschlungenen Körperteil zurückgezogen. Dabei streckt sich der Schlauch wieder und nimmt den Faden 8 um den zu umschlingenden Körperteil mit herum. Der Faden kann dann in an sich bekannter Weise beispielsweise verknotet werden, so daß eine Ligatur erzeugt wird.

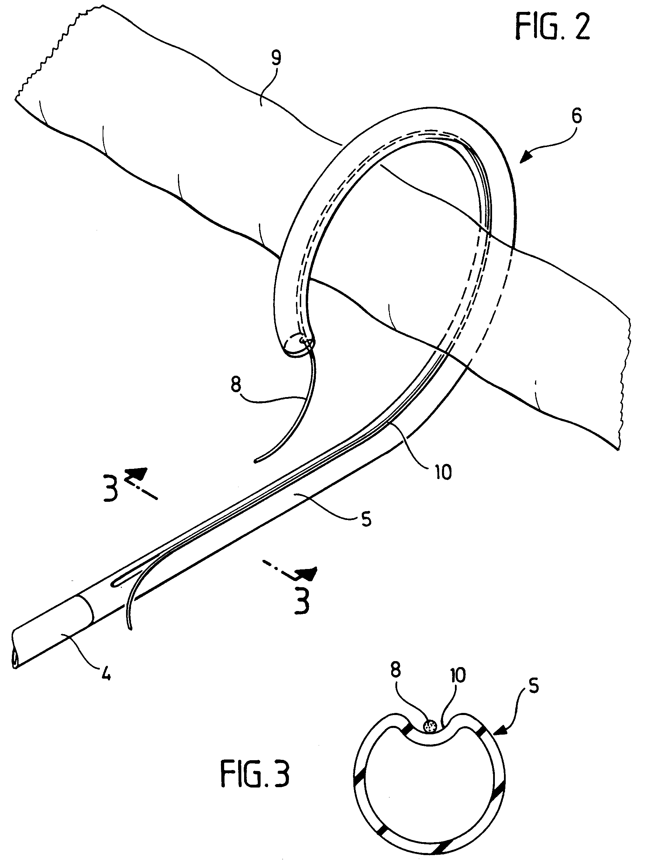

Während bei dem Ausführungsbeispiel der Figur 1 der Faden 8 frei in den Haken 7 eingehängt wird und sonst an dem Arm keinerlei Führung erfährt, ist der Faden 7 bei dem Ausführungsbeispiel der Figur 3 in einen Längskanal 10 eingelegt, der in die Außenwand des Armes 6 eingeformt ist. Die Einformung kann eine Einkerbung sein, wie dies in Figur 3 im Querschnitt dargestellt ist, vorzugsweise ist dabei die Einkerbung so stark gewählt, daß eine Einstülpung entsteht, so daß ein in diesen Längskanal 10 eingelegter Faden 8 in diesem Kanal festgelegt wird. Beim Umschlingen des Blutgefäßes 9 wird damit der Faden 8 mit dem Arm 6 um das Blutgefäß 9 herumgelegt und kann anschließend durch Zug an den Enden des Fadens 8 aus dem einseitig offenen Längskanal 10 wieder herausgezogen werden.

Bei einem anderen in der Zeichnung nicht dargestellten Ausführungsbeispiel könnte ein entsprechender Kanal auch im Innern des Armes 6 verlaufen, so daß bei einem Zurückziehen des Armes 6 der Faden in Umschlingungslage liegenbleibt.

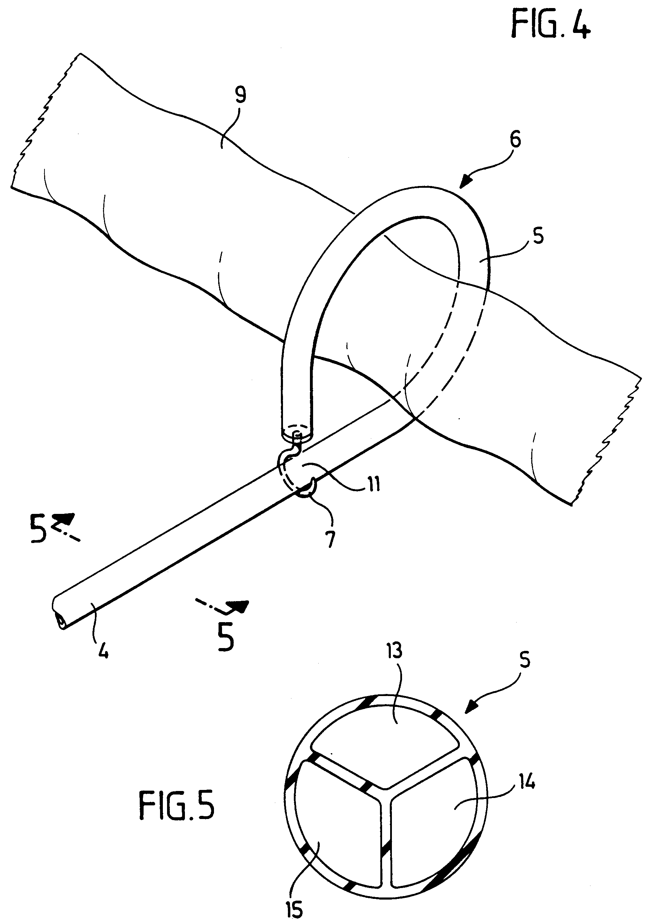

Das in Figur 4 dargestellte Instrument ist im wesentlichen gleich aufgebaut wie das der Figur 1, der Haken 7 ist jedoch so dimensioniert, daß er um einen rückwärtigen Bereich 11 des Armes 6 herumgelegt werden kann, so daß sich eine geschlossene Schlinge 12 des Armes 6 ausbildet. Das Einlegen des rückwärtigen Bereiches 11 kann dabei dadurch erfolgen, daß dieser rückwärtige Bereich 11 quer zur Ebene der Schlinge 12 bewegt und daher seitlich an den Haken 7 herangeführt wird. In gleicher Weise kann eine Lösung der Schlinge erfolgen.

Die Schlinge 12 kann durch Zug am Schlauch 5 zugezogen werden, sobald der Schlauch 5 wieder geleert und dadurch in seinen biegbaren Zustand überführt wird, so daß der Schlauch bei dieser Ausführungsform unmittelbar als Ligaturelement eingesetzt werden kann.

Bei einer bevorzugten Ausführungsform des Instrumentes wird eine Querschnittsform des Schlauches verwendet, wie sie in Figur 5 dargestellt ist. Der Schlauch umfaßt in diesem Falle drei in Umfangssrichtung versetzte, voneinander getrennte Kammern 13, 14, 15, die jeweils mit einer eigenen Flüssigkeitsquelle verbunden sind, also beispielsweise jeweils mit einem eigenen Spritzenkörper 1. Durch unterschiedliche Befüllung der drei Kammern 13, 14, 15 kann der Schlauch 5 in allen Richtungen gezielt abgebogen werden, beispielsweise auch quer zur Ebene der Schlinge 12. Dadurch wird es möglich, ohne Bewegung des Bereiches 11 den Haken 7 seitlich um diesen Bereich 11 herumzulegen und eine geschlossene Schlinge 12 auszubilden, bzw. eine derartige Schlinge nach Anwendung wieder zu lösen.

## Patentansprüche

1. Umfahrungsinstrument mit einem um einen Gegenstand herumlegbaren, länglichen, streckbaren und abbiegbaren Arm (6), an dessen freiem Ende ein Haltewerkzeug angeordnet ist, dadurch gekennzeichnet, daß der Arm (6) mindestens über einen Teil seiner Länge als Schlauch (5) aus einem dehnbaren Material ausgebildet ist, dessen Längsdehnbarkeit auf zwei einander gegenüberliegenden Seiten verschieden ist, daß das Innere des Schlauches (5) mit einer ein fließfähiges Medium unter Druck in den Schlauch (5) einführenden Druckquelle (1) verbunden ist und daß der Arm (6) im schlauchförmigen Bereich mindestens um 180° abbiegbar ist.

2. Instrument nach Anspruch 1, dadurch gekennzeichnet, daß das Haltewerkzeug ein Fadenhalter (7) ist.

3. Instrument nach Anspruch 2, dadurch gekennzeichnet, daß der Fadenhalter (7) ein Haken ist.

4. Instrument nach einem der voranstehenden Ansprüche, dadurch gekennzeichnet, daß längs des Armes (6) ein Fadenkanal (10) angeordnet ist.

5. Instrument nach Anspruch 4, dadurch gekennzeichnet, daß der Fadenkanal im Innern des Armes (6) angeordnet ist.

6. Instrument nach Anspruch 4, dadurch gekennzeichnet, daß der Fadenkanal (10) durch ein Rinne oder Einstülpung in der Wand des Armes (6) gebildet wird.

7. Instrument nach einem der voranstehenden Ansprüche, dadurch gekennzeichnet, daß der Arm (6) so weit abbiegbar ist, daß sein freies Ende den rückwärtigen Bereich (11) des Armes (6) erreicht.

8. Instrument nach Anspruch 7, dadurch gekennzeichnet, daß das Haltewerkzeug (7) ein Haken ist, der so dimensioniert ist, daß er um einen rückwärtigen Bereich (11) des Armes (6) legbar ist.

9. Instrument nach Anspruch 8, dadurch gekennzeichnet, daß im Innern des Schlauches (5) des Armes (6) mehrere getrennt befüllbare Kammern (13, 14, 15) angeordnet sind, so daß durch deren gezielte Befüllung ein Abbiegen des Schlauches (5) nach verschiedenen Richtungen möglich ist.

## Claims

1. A looping instrument comprising an elongate, extensible and bendable arm (6) placeable round an object, a holding tool being arranged on the free end of the arm (6), characterised in that the arm (6) is formed over at least part of its length as a tube (5) of extensible material, the linear extensibility of which is different on two opposing sides, in that the interior of the tube (5) is connected to a pressure source (1) introducing a flowable medium under pressure into the tube (5), and in that the arm (6) is bendable through at least 180° in the region formed as a tube.

2. An instrument according to claim 1, characterised in that the holding tool is a thread holder (7).

3. An instrument according to claim 2, characterised in that the thread holder (7) is a hook.

4. An instrument according to any one of the preceding claims, characterised in that a thread channel (10) is arranged along the arm (6).

5. An instrument according to claim 4, characterised in that the thread channel is arranged inside the arm (6).

6. An instrument according to claim 4, characterised in that the thread channel (10) is formed by a groove or depression in the wall of the arm (6).

7. An instrument according to any one of the preceding claims, characterised in that the arm (6) is bendable so far that its free end reaches the rear region (11) of the arm (6).

8. An instrument according to claim 7, characterised in that the holding tool (7) is a hook dimensioned so as to be placeable round a rear region (11) of the arm (6).

9. An instrument according to claim 8, characterised in that a plurality of separately fillable chambers (13, 14, 15) are arranged inside the tube (5) of the arm (6) so that it is possible to bend the tube (5) in different directions according to the chamber filled.

## Revendications

1. Instrument de contournement comportant un bras tout en longueur (6), qui peut s'étirer et se cintrer et se placer autour d'un objet et à l'extrémité libre duquel est disposé un outil de maintien, caractérisé par le fait que le bras (6) est conçu, au moins sur une partie de sa longueur, sous forme de tuyau flexible (5) en un matériau qui peut s'allonger et dont le coefficient d'allongement longitudinal est différent sur deux côtés opposés l'un à l'autre, que l'intérieur du tuyau flexible (5) est relié à une source de pression (1) qui introduit sous pression dans le tuyau (5) une masse fluide et que le bras (6) peut se cintrer sur au moins 180° dans la zone en forme de tuyau flexible.

2. Instrument selon la revendication 1, caractérisé par le fait que l'outil de maintien est un porte-fil (7).

3. Instrument selon la revendication 2, caractérisé par le fait que le porte-fil (7) est un crochet.

4. Instrument selon l'une des revendication précédentes, caractérisé par le fait que le long du bras (6) est disposé un canal à fil (10).

5. Instrument selon la revendication 4, caractérisé par le fait que le canal à fil est disposé à l'intérieur du bras (6).

6. Instrument selon la revendication 4, caractérisé par le fait que le canal à fil (10) est formé par une rigole ou un encastrement dans la paroi du bras (6).

7. Instrument selon l'une des revendication précédentes, caractérisé par le fait que le bras (6) peut se cintrer suffisamment pour que son extrémité libre atteigne la zone arrière (11) du bras (6).

8. Instrument selon la revendication 7, caractérisé par le fait que l'outil de maintien (7) est un crochet qui est dimensionné de façon à pouvoir se placer autour d'une zone arrière (11) du bras (6).

9. Instrument selon la revendication 8, caractérisé par le fait qu'à l'intérieur du tuyau flexible (5) du bras (6) sont disposées plusieurs chambres (13, 14, 15) que l'on peut remplir séparément de sorte que leur remplissage spécifique rend possible un cintrage du tuyau flexible (5) selon différentes directions.
